(19) Europäisches Patentamt
European Patent Office
Office européen des brevets

(11) Publication number : 0 390 040 B1

(12) # EUROPEAN PATENT SPECIFICATION

(45) Date of publication of patent specification : **26.05.93 Bulletin 93/21**

(51) Int. Cl.$^5$ : **C07K 15/14**, A61K 35/78

(21) Application number : **90105725.7**

(22) Date of filing : **26.03.90**

(54) **Ribosome inactivating proteins and derivatives thereof.**

Consolidated with 90904825.8/0465495 (European application No./publication No.) by decision dated 04.06.92.

(30) Priority : **31.03.89 IT 1996489**

(43) Date of publication of application : **03.10.90 Bulletin 90/40**

(45) Publication of the grant of the patent : **26.05.93 Bulletin 93/21**

(84) Designated Contracting States : **AT BE CH DE DK ES FR GB GR IT LI LU NL SE**

(56) References cited :
**WO-A-88/09123**
**BIOLOGICAL ABSTRACTS, vol. 77, no. 12, 1984, abstract no. 94736, Philadelphia, PA, US; F. STIRPE et al.: "Ribosome-inactivating proteins from the seed of Saponaria officinalis (soapwort), of Agrostemma githago (corn cockle) and of Asparagus officinalis (asparagus), and from the latex of Hura crepitans (sandbox tree)", & BIOCHEM. J. 216(3): 617-626, 1983**
**CHEMICAL ABSTRACTS, vol. 100, no. 19, 7th May 1984, pages 19-20, abstract no. 150703d, Columbus, Ohio, US; F. STIRPE et al.: "Ribosome-inactivating proteins as possible chemotherapeutic agents", & FORTSCHR. ONKOL. 1983, 10 142-6**
**CHEMICAL ABSTRACTS, vol. 105, no. 25, 22nd December 1986, page 253, abstract no. 220636p, Columbus, Ohio, US; F. STIRPE et al.: "Bryodin, a ribosome-inactivating protein from the roots of Bryonia dioica L. (white bryony)", & BIOCHEM. J. 1986, 240(3), 659-65**
**CHEMICAL ABSTRACTS, vol. 112, no. 9, 26th February 1990, page 486, abstract no. 73912q, Columbus, Ohio, US; T.B. NG et al.: "Investigation of ribosome inactivating protein-like activity in tissues of Cucurbitaceae plants", & INT. J. BIOCHEM. 1989, 21(12), 1353-8**

(56) References cited :
**CHEMICAL ABSTRACTS, vol. 112, no. 9, 26th February 1990, page 369, abstract no. 72697m, Columbus, Ohio, US; A. BOLOGNESI et al.: "Purification and properties of a new ribosome-inactivating protein with RNA N-glycosidase activity suitable for immunotoxin preparation from the seeds of Momordica cochinchinensis", & BIOCHIM. BIOPHYS. ACTA 1989, 993(2-3), 287-92**
**A Falasca et al.: Biochem. J. (1982), 207,505-509**

(73) Proprietor : **ITALFARMACO S.p.A.**
**Viale Fulvio Testi, 330**
**I-20126 Milan (IT)**

(72) Inventor : **Stirpe, Fiorenzo**
**Via Carducci 125**
**20099 SESTO S. GIOVANNI (MILANO) (IT)**
Inventor : **Barbieri, Luigi**
**Via Carducci 125**
**20099 SESTO S. GIOVANNI (MILANO) (IT)**
Inventor : **Gromo, Gianni**
**Viale Fulvio Testi, 330**
**I-20126 Milano (IT)**

(74) Representative : **Minoja, Fabrizio**
**Studio Consulenza Brevettuale Via Rossini, 8**
**I-20122 Milano (IT)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention refers to ribosome inactivating proteins and their sulphydrylated derivatives useful for the preparation of immunotoxins.

Ribosome-inactivating proteins from plants (RIPs) (Cancer Surveys 1, 489, 1982; J. Natural Products. 48, 446, 1985, FEBS Letters 195, 1, 1986) catalytically inactivate the 60 S ribosomal subunits of eukaryotic ribosomes. They can be either single-chain proteins (RIPs type 1) or two-chain proteins (RIPs type 2), in which one chain has the enzymic activity and the other one has the properties of a galactose-specific lectin. RIPs type 1 are more frequent, being present in several parts of many and possibly all plants, including seeds, roots, leaves and latices, sometimes in more than one form, possibly isoforms. RIPs have an unusual N-glycosidase activity, and cleave the N-glycosidic bond of adenine$^{4324}$ of 28S rRNA introducing a lesion that renders RNA cleavable by aniline at a site adjacent to the cleavage induced by $\alpha$-sarcin. This makes ribosomes unable to bind the elongation factors 1 or 2, with consequent arrest of protein synthesis. In spite of the numerous similarities in their structure and of the identical enzymic activity, RIPs have different effects on ribosomes from different organisms (from plants, protozoa, insects and other metazoa).

Interest in RIPs is growing since they have been used as components of "immunotoxins", hybrid molecules consisting of a toxic moiety linked to an antibody. Hopefully immunotoxins will be useful to eliminate harmful cells, neoplastic, immunocompetent and parasitic cells being considered as possible targets (Cancer Immunol. Immunother. 27, 95, 1988).

Additional interest on RIPs comes from their antiviral activity. All RIPs tested inhibited the infectivity of plant and animal viruses. Actually the first identified RIP type 1, namely the pokeweed antiviral protein (PAP), was initially purified as an anti-viral protein. This property of RIPs was attributed to an easier penetration into virally infected cells with consequent inactivation of their ribosomes and arrest of viral multiplication. Recently, however, a RIP type 1, trichosanthin, was found to inhibit replication of human immunodeficiency virus through a mechanism apparently independent of the effect on ribosomes (..).

It is useful to have several RIPs available for the preparation of immunotoxins, to have conjugates active against cells resistant to some RIPs, and to circumvent the problems arising from the neutralization due to the immunological reaction after administration and from the inactivation of some RIPs during the conjugation procedure.

It has now been found new ribosome inactivating proteins obtainable from the plants Momordica cochinchinensis, Bryonia dioica e Asparagus officinalis. Said plants were already previously studied without recognizing therein the presence of the proteins of the invention, which are characterized by a remarkably higher activity than that of known similar proteins, extracted from the same vegetal species. For instance, the protein extracted from Momordica cochinchinensis, a cucurbitacea of indian origin, exhibit surprisingly different characteristics than the known momordine, extracted from the some Momordica genus (Momordica charantia). Analogously, three glycoproteins are known from Asparagus officinalis L. (Biochem. J. 216, 617, 1983) whereas the substances of the invention extracted from the same species are proteins having different biological and chemico-physical characteristics. The activity of the proteins of the invention consists in a potent inhibition of protein synthesis, mainly in cell-free systems: although poorly toxic to most mammalian cells, they may be converted into highly cytotoxic agents by conjugation - with suitable chemical agents - to suitable carriers ("haptomers"), e.g. monoclonal antibodies. If said carriers are antigen-specific for tumoral cells, said new RIPs may be used for the selective destruction of the latter.

It has also been found that the proteins of the invention can inhibit the virus replication in cultured cells acutely infected with different viruses including the human immunodeficiency virus (HIV 1).

The invention concerns also the derivatives of said RIPs wherein sulphydryl groups were introduced, represented by the formula I. Said derivatives have the same or slightly lower inhibitory activity than the starting RIPs and therefore, since they are both the synthesis intermediates of immunotoxins and the inhibiting agents that the latter release into the cells, they are useful for the preparation of antibody-toxins conjugates.

$$\text{Tox}---\left[---\text{Y}---\text{S}---\text{R}\right]_{x}$$

(I)

(wherein Tox - represents the considered RIP; Y, R and x have the meanings hereinbelow reported)

The derivatives of formula I are obtained according to conventional methods using bifunctional reagents such as, for instance, dimethyl 3,3'-dithiobispropionimidate or the reagents hereinbelow reported: N-succinimidyl-3-(2-pyridyldithio)propionate (SPDP)

Y: 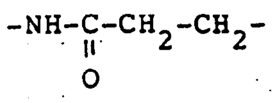 R: 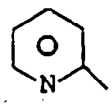

x: 0,5-3 (preferably 0,7-2, most preferably 0,7-1,5)

2) 2-Iminothiolane (Traut's reagent):

Y: 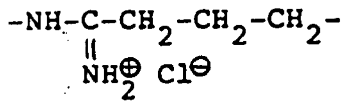

R: H x: 0,5-2 (preferably 0,5-1, even more preferably 0,7)

3) S-acetyl-mercapto-succinic anhydride (SAMSA):

Y:

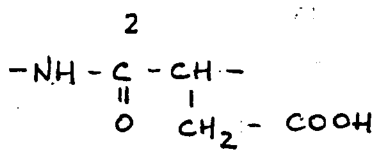

R: H; $CH_3$-CO- x: 0,2-3(preferably 0,4-1, most preferably 0,7-1)

The RIPs of the invention,will be designed'with the following names: momorcochin (from Momordica cochinchinensis seeds); bryodine-L (from Bryonia dioica leaves); asparin 1 and asparin 2 (from Asparagus officinalis seeds). Momorcochin and bryodin-L are glycoproteins, whereas asparin 1 and 2 are proteins.

The proteins of the invention, whose characteristics are hereinafter reported, are obtained by conventional methods for the extraction of proteins from plants. The proteins of the invention are therefore obtainable by a process comprising:

a) grinding and extraction of seeds or leaves;

b) centrifugation or filtration of the extract;

c) chromatographies on cross-linked dextrane or dialysis of the surnatant of the step b)

d) ion-exchange chromatography and/or gel filtrations in suitable sequences;

e) final purification by means of dialysis, lyophilization, chromatography, precipitation or other usual methods.

The so obtained proteins may be modified by means of the previously cited reagents, according to known methods. The obtained derivative may then be used for the preparation of immunotoxins which may be used in therapy, for instance for the treatment of tumoral forms in which case they will be administered by parenteral route in doses ranging from 1 to 100 mg, using suitable pharmaceutical formulations.

The following examples further illustrate the invention.

## EXAMPLE 1

Preparation of crude extracts

The seeds are wet-ground, in suited grinders and extracted; leaves and roots, sliced in small pieces are subjected to centrifugation with filtration before extraction, recovering the juice and extracting the residue. The extraction is carried out with a 0.14 M sodium chloride/5mM sodium phosphate buffer, pH 7.4-7.5, under stirring for 12 hours at the temperature of 4-10°C.

Clarification is carried out by centrifugation; for leaves and roots the corresponding juices, obtained as above described, are added to the clarified mixture and then clarified again by high g centrifugation. The final clarified solutions ("crude extracts") are subjected to the following phase. The extracts are adjusted to pH 4.2-4.8, e.g. to pH 4.5, with acetic acid; after standing, a precipitate is formed which is centrifugated and discarded. The clear supernatants ("acidic extracts") are charged on a S-sepharose$^R$ column, equilibrated with 10mM sodium acetate buffer, pH 4.5. After washing with the same buffer and then with 5mM sodium phosphate, pH 7.5, till neutrality of the effluent, the column is eluted with 1M sodium chloride/phosphate buffer.

The clear eluates are saturated with ammonium sulfate; after standing, the precipitates are collected by centrifugation.

The solids are dissolved in 5mM phosphate buffer, pH 7.0; the solutions are clarified by centrifugation, and subjected to the isolation step of the single RIPs ("S-Sepharose percolate"). Alternatively, said final extract may be prepared by subjecting the crude extracts to dialysis for at least 24 hours, at 4°C, against 50-500 volumes of 5mM phosphate buffer, pH 6.5, with at least two total changes. Each precipitate formed during the dialysis is removed by centrifugation and the clear extract is then subjected to the isolation procedure. During the steps of: "crude extract", "acidic extract", "S-Sepharose percolate", are carried out determinations of total proteins content, specific and total activity, hereinafter described for each RIP.

## EXAMPLE 2

Isolation and characterization of momorcochin

A) Extraction and isolation

1.2 kg of Momordica cochinchinensis seeds, a Cucurbitaceae species of indian origin, are extracted as disclosed in Example 1. The final solution "S-Sepharose percolate" is chromatographed on Sephadex G-50$^R$ ("Sephadex G-50 eluate") and then charged on a CM-Sepharose Fast Flow$^R$ column, equilibrated with 5mM sodium phosphate buffer, pH 7.0, and provided with flow-cell and absorption detector at 280 nm. The column is washed with the equilibration buffer until the percolate does not absorb any longer at 280 nm. The column is eluted with NaCl, in linear gradient from 0 to 300mM, in the same buffer, with percolation rate of 1.2 l/h, at 20°C, for a total volume of 20 l, collecting fractions of 450 ml. Each fraction is assayed for both the absorption at 260 nm and conductimetry (mS:cm). Each fraction is also assayed for the activity inhibiting specific and total protein synthesis, as hereinafter reported in the biological characteristics.
The comparison between the elution pattern and that known for momordin (from Momordica charantia: J. Chromatogr. 408, 235, 1987) confirms the non coincidence of the two substances (momordin: elution fractions from 12 to 17 l about, momorcochin: fractions from 6 to 7.5 l). The momorcochin-containing fractions were pooled and dialyzed against water until complete salting-out. The final solution is lyophilized and the residue ("momorcochin") is analyzed for chemico-physical and biological characteristics.

B) Chemico-physical characterization

a) Protein content of the extraction solutions: results obtained according to the methods:
- by Lowry (J. Biol. Chem. 193, 265, 1951) (standard: bovine serum albumin),
- by Kalb & Bernlhor (Anal. Biochem. 82, 362, 1977),
- spectrophotometric, using the absorption at 280nm of the purified protein, with molar extinction coefficient of 0,8.

b) Relative molecular mass (Mr):
determined by:
- electrophoresis on polyacrylamide gel (Nature 227, 680, 1970) with the following markers (between brackets the relative Mr): cytochrome C (12300), myoglobin (17200), carbonic anhydrase (30000), ovoalbumin (45000), bovine serum albumin (66250), ovotransferrin (76000-78000);

- gel-filtration through Sephacryl S-200R (95 x 1.6 cm columns) equilibrated with 20mM sodium phosphate buffer, pH 7.5 containing 0.3M NaCl; elution rate 8 ml/h at 20°C; calibration (between brackets the relative Mr): dextran blue (2 x $10^6$), bovine serum albumin (66250), ovoalbumin (45000), chymotrypsinogen A (25000), ribonuclease A (13700).

c) Isoelectric point, amino acid composition, amino sugar and neutral sugar compositions; determined according to Biochem. J. 240, 659, 1986.

By applying said methods to the intermediate extracts (described in Example 1) to those of this Example and to the final lyophilized product "momorcochin", the following results are obtained:

Proteins at different purification steps (g/l of kg starting seeds):

- "crude extract" 52.26
- "acidic extract" 36.00
- "S-Sepharose percolate" 19.48
- "Sephadex G-50 eluate" 2.55
- "momorcochin" 0.74

i.e. a yield of about 1% by weight in momorcochin relative to the total proteins first extracted and 0.07% by weight relative to the extracted seeds.

Relative molecular mass of the final product:

by gel filtration: 31000
by electrophoresis: 30700

Electrophoresis shows also that a single chemical species is present.

Isoelectric point > 9 (momordin: 8.60 - Biochem. J. 207, 505, 1982)
Extinction at 280 nm (A280): 0.7
Amino acid compositions (mols/mole protein)
(means values from hydrolysis at 24, 48 and 72 h; ratios calculated assuming Mr=30700; error < 1%; IUPAC symbols, between brackets the corresponding values found for momordin - Biochem. J. 207, 505, 1982; aa: amino acid):

| aa | mol/mol pr. | aa | mol/mol pr. | aa | mol/mol pr. |
|---|---|---|---|---|---|
| Lys | 15.1 (11.4) | Glx | 21.7 (24.8) | Met | 3.3 ( 7.0) |
| His | 2.7 ( 4.7) | Pro | 8.8 ( 9.0) | Ile | 12.3 (13.8) |
| Arg | 8.1 (12.6) | Gly | 11.3 (15.8) | Leu | 26.6 (21.2) |
| Asx | 27.7 (27.6) | Ala | 23.6 (24.6) | Tyr | 11.4 (13.4) |
| Thr | 18.4 (16.2) | Half-Cys | abs. (2.2) | Phe | 10.8 ( 8.4) |
| Ser | 19.2 (16.8) | Val | 18.7 (13.7) | Trp | abs. ( 1.0) |

The comparison with the composition of momordin shows the different compositions of the two RIPs, extracted from two species of the same genus Momordica.

Total content in neutral sugars: 2.82%
(Momordin - Biochem. J. 207, above cited - has a content of 1.74%).
Sugar composition (mols/mol RIP):
(ratios calculated assuming rM: 30700, IUPAC symbols; between brackets the corresponding values for momordin - Biochem. J. 207, above cited):

| Sugars | | mols/mol RIP |
|---|---|---|
| Fuc | : | 1.42 (0.90) |
| Glc | : | 0.98 (0.80) |
| Man | : | 2.16 (1.30) |
| Xyl | : | 0.98 (0.50) |
| $Glc\text{-}NH_2$ | : | abs. (2.00) |

Also in this case the unexpected difference between momorcochin and momordin is evident.

C) Biological characterization

C.1 - Inhibiting activity on protein synthesis: assayed by:

C.1.1 - Rabbit reticulocyte lysate (Biochem. J. 240, 659, 1986). By this method, the ability of RIPs to inhibit the protein synthesis in the presence of reticulocyte components (post-mitochondrial fraction) is determined.

The method is based on the "in vitro" induction of the synthesis with said sub-cellular preparation, in the presence of suitable cofactors and of the mixture of natural amino acids one of which is labelled with 3H or 14C. The amount of incorporated radioactivity in the protein obtained, in function of time, allows to evaluate the protein synthesis rate of the system and the effects of different agents, such as RIPs, thereon.

10mM Tris/HCl buffer solutions, pH 7.4, containing 100mM ammonium acetate, 2mM magnesium acetate, 1mm AT, 0.2mM GTP, 15mM phosphocreatine, 0.5mM natural amino acids (excluding leucine), are added with: 3µg creatine kinase, 89nCi of L-(14C)-leucine and 25 µl of rabbit reticulocyte lysate (prepared according to J. Biol. Chem. 237, 760, 1982), the final volumes being 62.5 µl. After incubation at 28°C for 5' the reaction is stopped by addition of 1 ml of 0.1 M KOH. The measurement of the incorporated radioactivity is carried out as disclosed in Biochem. J. 174, 491, 1978. Scalar amounts of RIP are added before the incubation under the same experimental conditions. The results are expressed as $IC_{50}$ (concentration inhibiting by 50% the synthesis) or in units and specific activities (units/mg). An inhibition unit (or activity unit), the amount (in mg) of RIP, needed for inhibiting by 50% the protein synthesis, adjusting the reaction volume to 1 ml.

C.1.2 - Polyuridylate (poly-U)-directed Polymerization - with ribosomes from rabbit or Trypanosoma brucei reticulocytes - of phenylalanine (Proc. Natl. Acad. Sci. U.S. 47, 1588, 1961). This method allows the evaluation of the inhibiting activity of the RIPs on the polyphenylalanine synthesis, directed by the synthetic polynucleotide poly-U, consisting only of the uracil nucleotide, starting from t-RNA esterified phenylalanine.

The method is similar to the previous one but, instead of rabbit reticulocyte mRNAs - directing the synthesis of complex proteins - a simplified polynucleotide is used which translates the synthesis of an homopolymer, polyphenylalanine, in the presence of purified ribosomes.

An 80mM Tris/HCl buffer, pH 7.4, at a final volume of 250µl containing 120mM magnesium acetate, 2mM GTP, is added with: 200µg poly-U, 25 pmol of 14C-phenylalanyl-t-RNA, 20 pmol of rabbit reticulocyte ribosomes or Trypanosoma brucei ribosomes (J. Protozool. 35, 384, 1988), 250µg (as proteins) of "pH 5 supernatant", according to Biochem. J. 176, 265, 1978). After incubation at 30°C for 30', the trichloroacetic acid-insoluble-radioactivity (i.e. the amount of polymerized labelled phenylalanine) is measured (Biochem. J. 240, cited). The experiment is then carried out again in the presence of scalar amounts of RIPs. The $IC_{50}$ is calculated by linear-regression analysis.

C.1.3 - Human cell cultures: HeLa, TG cells (human oviduct carcinoma, Bourgeois C.A., Hemon, D. & Bouteille, M. (1979) Structural relationship between the nucleolus and nuclear envelope. J. Ultrastruct. Res. 68, 328.), JAR cells (human choriocarcinoma, Pattillo, R.A., Rucker, A., Hussa, R., Berstein, R. & Delfs, E. (1971) The Jar cell line continuous human multihormone production and control. In Vitro 6, 398.), human fibroblasts, and NB100 cells (human neuroblastoma cell line obtained by biopsy from hospitalized patients), were maintained as monolayer cultures in RPMI 1640 medium supplemented with non-essential amino acids, antibiotics and 10% foetal calf serum. This method allows the evaluation of the cytotoxic effect of RIPs in different integer cell systems, by comparing the protein synthesis in the presence and in the absence of toxins.

The method consists in culturing different cells, in RPMI 1640 medium free from foetal calf serum, in the presence of different amounts of RIPs. After a period of 18 hours, the activity of protein synthesis is measured by incubating the cells for 1-5 hours in an RPMI 1640 medium, free from serum and leucine and added with 0.1-1 μCi of L-(14C)-leucine/ml. The radioactivity incorporated in the cells is measured according to J. Biol. Chem. 257, 7495, 1982. The $ID_{50}$ (dose inhibiting 50% of protein synthesis) is calculated by linear-regression analysis.

C.2 - Toxicity in animals

The toxicity of pure RIPs was evaluated in Swiss female mice weighing 27-32 g, fed ad libitum, by i.p. route at 6 different doses ranging from 5.6 to 23.7 mg/kg body weight, 6 animals per dose. The $LD_{50}$ was calculated 48 hours after by linear-regression analysis. The autoptic examination was carried out on the main organs.

By applying said methods to the intermediate extracts of Example 1, to those of the present Example and to the final lyophilized product, ("momorcochin"), the following results are obtained:

Inhibiting activity of protein synthesis with rabbit reticulocyte lysate (C.1.1.):

| Fraction | Specific Activity (unit/mg x $10^{-3}$) | Extracted Total Activity* (unit x $10^{-6}$) | Yield** |
|---|---|---|---|
| -"crude extract" | 16.8 | 877.2 | (100) |
| -"acidic extract" | 29.1 | 1,049.6 | 119.6 |
| -"S-Sepharose percolate" | 27.5 | 536.6 | 61.2 |
| -"Sephadex G-50 eluate" | 149.2 | 379.1 | 43.2 |
| -"momorcochin" | 285.7 | 211.9 | 24.0 |

* : referred to 1.2 kg of starting seeds

** : referred to the "crude extract", assumed as 100.

It has to be pointed out that the specific activity of momorcochin (211.9 units/mg x $10^{-3}$ is about 201 times higher than that of momordin (reported to be 1.0526 x $10^{-3}$ units/mg in J. Chromatogr. 408, 235, 1987).

IC50 for the protein synthesis in cell-free systems

| Method | Enzymatic systems | IC50 (nM) |
|---|---|---|
| C.1.1 | Reticulocyte lysate | 0.12 |
| C.1.2 | Purified ribosomes from reticulocytes | 1.0 |
| C.1.2 | Purified ribosomes from T. Brucei | 3,330. |

Comparing the $IC_{50}$ for momordin (FEBS-Letters 195, 1, 1986) obtained with reticulocyte lysate (0.06nM) with that of momorcochin, a further differentiation between the two RIPs is evident.

IC50 for the protein synthesis in integer cells (C.1.3):

(The values are the mean of two different determinations; between brackets the $ID_{50}$ for momordin; symbols disclosed in item C.1.3):

| Cell | ID50 (nM) |
|---|---|
| HeLa | 2,870 (32,000) |
| TG | 2,040 |
| Jar | 3,330 |
| NB100 | 320 |
| Human fibroblasts | 109 |

Toxicity in animals (C.2): LD50: 24.5 mg/kg body weight. Momordin has a $LD_{50}$ of 4.30 mg/kg body weight (FEBS Letter, above cited).

D) Chemical modifications

D.1 - With SPDP: the method described in Biochem J. 240, 659, 1986 is used. The determination of introduced 2-pyridylsulphydryl groups is carried out according to Biochem. J. 173, 727, 1978. Momorcochin to be sulphydrylated is dissolved in borate buffer, pH 9.0, at the concentration of 10 mg/ml. The molar ratio of SPDP to momorcochin, the number of introduced groups, and the $IC_{50}$ are reported in the table D.4.

D.2 - With 2-iminothiolane: the method described in Biochemistry 24, 1517, 1985 is used. The number of introduced sulphydryl groups is determined, according to Arch. Biochem. Biophys 82, 70, 1959. Momorcochin is in 1.5 mM solution in 50 mM borate buffer, pH 9.0. The purification of the final product requires a gel-filtration through Sephadex G 25. The molar ratio of 2-iminothiolane to momorcochin and the $IC_{50}$ are shown in Table D.4.

D.3 - With SAMSA: according to J. Am. Chem. Soc. 81, 3802, 1959. The number of introduced sulphydrhyl groups is determined, after addition of hydroxylamine, according to Methods in Enzymology 25, 457n 1972. Momorcochin is in solution of 125mM phosphate buffer pH 7.

D.4 - Determination of IC50 of modified RIPs

The IC50 values of the modified products are calculated with reticulocyte lysate (Method C.1.1) and are expressed in nM. Each value is the mean of three determinations.

| Reagent | Molar ratio Reagent/RIP(*) | Number of introduced groups | IC50 (nM) |
|---|---|---|---|
| None | | | 0.12 |
| SPDP | 1.5 : 1 | 1.13 | 0.12 |
| SPDP | 2.0 : 1 | 1.44 | 0.20 |
| 2-Iminothiolane | 1.25 mM | 0.73 | 0.10 |
| SAMSA | 28 : 1 | 0.70 | 0.10 |

(*): under the above reported experimental conditions

## EXAMPLE 3

Isolation and characterization of bryodine -L

A) Extraction and isolation

12.5 kg of fresh leaves of Bryonia dioica, a cucurbitacea species, are extracted as in Example 1 up to the obtaining of "S-Sepharose percolate"; said solution is worked up as in Example 2, using, instead of Sephadex G-50$^R$, Sephacryl-S 200$^R$ ("Sephacryl-S200 eluate"); after chromatography on CM-Sepharose Fast Flow$^R$, under the conditions described for momorcochin, the active fractions ("CM-Sepharose eluate"), are pooled, dialyzed extensively against water and charged on a Blue-Sepharose$^R$ column, equilibrated with 10mM Tris/HCl buffer, pH 8.0; the column is eluted with a 0-200mM NaCl gradient, in the same buffer, monitoring the absorption at 280 nm and the conductivity of the fractions (mS/cm).

The fractions from 6.8 to 7.6 1 are pooled and dialyzed extensively against water and then lyophilized (bryodine-L).

B) Chemico-physical characterization.

The used methods are those disclosed in B of Example 2, paragraphs a, b, c.

Proteins at different purification steps (g/12.5 kg of leaves).

- "crude extract" 293.75
- "S-Sepharose percolate" 1.74
- "Sephacryl-S 200 eluate" 0.41
- "CM-Sepharose eluate" 0.035
- "Bryodine-L" 0.021

Relative molecular mass of the final product:

by gel-filtration : 27300
by electrophoresis : 28800
The electrophoresis further shows that the lyophilized is a single substance.
Isoelectric point: > 9.5
Extinction at 280 nm (A280): 0.8

Aminoacid composition (mols/mol protein)
(mean values of the hydrolysis at 24, 48, 72 hours; ratios calculated assuming rM = 28800, error < 1%; IUPAC symbols; between backets the corresponding values found for bryodine - Biochem. J. 240, 659, 1986; aa: amino acid).

| aa | mol/mol pr. | aa | mol/mol pr. | aa | mol/mol pr. |
|---|---|---|---|---|---|
| Lys | : 10.8 ( 8.6) | Glx | : 18.9 (17.7) | Met | : 2.2 ( 1.6) |
| His | : 1.0 ( 1.9) | Pro | : 7.2 ( 6.7) | Ile | : 15.4 (15.1) |
| Arg | : 11.0 (11.8) | Gly | : 11.4 (11.4) | Leu | : 24.5 (28.3) |
| Asx | : 2 .5 (22.5) | Ama | : 24.1 (22.4) | Tyr | : 11.7 (14.2) |
| Thr | : 17.4 (15.1) | Half-Cys | :abs. (0.24) | Phe | : 7.4 ( 8.3) |
| Ser | : 24.4 (30.2) | Val | : 14.4 (15.6) | Trp | : abs. ( 2.0) |

The comparison between data shows the different compositions of the two RIPs. This difference is even more marked from the following data.

Total content in neutral sugars: 2.72%

For bryodine (Biochem. J. 240, cited) the total content is 6.33%.

Sugar compositions (mols/mol RIP):

(ratios calculated assuming rM : 28800 ; IUPAC symbols; between brackets the corresponding values for bryodine - Biochem. J. 240, cited):

| Sugars | | mols/mol RIP |
|---|---|---|
| Fuc | : | 1.52 (3.47) |
| Glc | : | 0.43 (1.55) |
| Man | : | 2.52 (6.27) |
| Xyl | : | 0.63 (0.93) |

C) Biological characterization

The biological characterization is assayed according to the methods described in Example 1, items C.1.1., C.1.2, C.1.3, whereas the toxicity is evaluated according to C.2.

Inhibiting activity of protein synthesis with rabbit reticulocyte lysate (C.1.1):

| Fraction | Specific Activity (unit/mg x $10^{-3}$) | Extracted Total Activity* (unit x $10^{-6}$) | Yield** |
|---|---|---|---|
| -"crude extract" | 0.6 | 170.0 | (100) |
| -"S-Sepharose percolate" | 81.4 | 141.0 | 83.2 |
| -"Sephacryl S-200 eluate" | 123.0 | 50.6 | 29.7 |
| -"CM-Sepharose eluate" | 251.9 | 8.7 | 5.1 |
| -"Bryodine-L" | 362.3 | 6.7 | 3.9 |

* : referred to 12.5 kg of fresh leaves

** : referred to "crude extract", assumed as 100.

It should be pointed out that the specific activity of bryodine-L (362.3 units/mg $10^{-3}$) is 2.8 and 1.6 times higher, respectively, than that of the CM 0.097 M and CM 0.112 M fractions, from leaves, and 2.56 times higher then bryodine (CM 0.100 M fraction, from roots) as reported in Biochem. J. 240, cited.

IC50 for protein synthesis in cell-free systems

| Method | Enzymatic systems | IC50 (nM) |
|---|---|---|
| C.1.1 | Rabbit reticulocyte lysate | 0.09 |
| C.1.2 | Purified ribosomes from reticulocytes | 1.3 |
| C.1.2 | Purified ribosomes from T. Brucei | 3,330. |

In this case the comparison with the data described in Biochem. J. 240 is not possible since either reported in incorporated d.p.m. or the $ID_{50}$ (with the same meaning of $IC_{50}$) was evaluated with different ribosome preparations (from wheat germs or from Bryonia dioica).

IC50 for protein synthesis in integer cell systems (C.1.3):

(the values are the mean of two different determinations)

| Cells | ID50 (nM) |
|---|---|
| HeLa | 3,330 |
| TG | 770 |
| Jar | 3,330 |
| NB100 | 50 |
| Human fibroblasts | 800 |

Toxicity in animals (C.2): LD50: 10 mg/kg body weight. Bryodine (Biochem. J. 240, cited): 14.5 mg/kg.

D) Chemical modifications

The chemical modifications are carried out using the methods D.1, D.2, D.3 of Example 2.

Determination of IC50 of modified bryodine-L:

The IC50 are determined according to the method C.1.1 of Example 2 and are expressed in nM. Each value is the mean of three determinations.

| Reagent | Molar ratio Reagent/RIP(*) | Number of introduced groups | IC50 (nM) |
|---|---|---|---|
| None | | | 0.10 |
| SPDP | 1.5 : 1 | 1.03 | 0.11 |
| SPDP | 2.0 : 1 | 1.32 | 0.12 |
| 2-Iminothiolane | 1.25 mM | 0.70 | 0.09 |
| SAMSA | 28 : 1 | 0.75 | 0.11 |

**EXAMPLE 4**

Isolation and characterization of asparin 1 and 2

A) Extraction and isolation

1 kg of Asparagus officinalis seeds is extracted as in Example 3, the CM-Sepharose extract is separated in two fractions ("CM-peak 1: fractions between 1.8 and 2.4 l; "CM-peak 2: fractions between 3.3 and 3.8 l). The two fractions are then purified separately on two Red-Sepharose$^R$ columns, equilibrated with Tris/HCl buffer, pH 8.0, eluting with a NaCl gradient 0-300 mM in the same buffer.

The purified fraction from "CM-peak 1" ("asparin 1") is lyophilized, wheras that from "CM-peak 2" ("asparin 2") is first eluted through a Sephacryl S-200$^R$ column.

B) Chemico-physical characterization

The methods of item B of Example 2, paragraphs a, b, c, were used.

Proteins at different purification steps (g/lkg seeds):

- "crude extract" 23.23
- "acidic extract" 13.58
- "S-Sepharose percolate" 7.55
- "Sephacryl-S 200 eluate" 2.04
- "CM-peak 1" 0.159
- "CM-peak 2" 0.229
- "asparin 1" 0.054
- "asparin 2" 0.049

Relative molecular mass of the final products:

Asparin 1:
by gel filtration : 29700
by electrophoresis : 30500
asparin 2:
by gel filtration : 28100
by electrophoresis : 29800
The electrophoresis shows also that the two fractions are single substances.

Isoelectric point: -asparin 1 : 8.7
-asparin 2 : 9.2
Extinction at 280 nm (A280): 1.0 (both)
Amino acid composition: (mol/mol protein)
asparin 1:
(mean values of hydrolysis at 24, 48, 72 hours; ratios calculated assuming Mr = 30500; error < 1%; IUPAC symbols; aa: amino acid):

| aa | mol/mol pr. | aa | mol/mol pr. | aa | mol/mol pr. |
|---|---|---|---|---|---|
| Lys | 15.3 | Glx | 24.3 | Met | 4.1 |
| His | 3.4 | Pro | 14.9 | Ile | 11.4 |
| Arg | 14.4 | Gly | 15.0 | Leu | 26.3 |
| Asx | 26.5 | Ala | 19.5 | Tyr | 9.8 |
| Thr | 13.9 | Half-Cys | 3.3 | Phe | 6.3 |
| Ser | 11.0 | Val | 16.7 | Trp | abs. |

Asparin 2:
(mean values of hydrolysis at 24, 48, 72 hours; ratios calculated assuming Mr = 29800; error < 1%; IUPAC symbols; aa: amino acid):

| aa | mol/mol pr. | aa | mol/mol pr. | aa | mol/mol pr. |
|---|---|---|---|---|---|
| Lys | : 14.9 | Glx | : 23.2 | Met | : 1.1 |
| His | : 2.9 | Pro | : 15.4 | Ile | : 11.5 |
| Arg | : 14.8 | Gly | : 15.2 | Leu | : 26.6 |
| Asx | : 27.2 | Ala | : 18.8 | Tyr | : 9.8 |
| Thr | : 14.2 | Half-Cys | : 1.3 | Phe | : 6.3 |
| Ser | : 11.3 | Val | : 16.9 | Trp | : abs. |

Total content in neutral sugars: 0%

For the three glycoproteins described (Biochem. J. 216, cited) the contents are: 1.42%, 1.20%, 1.32% with a composition:

| Sugar | Glycoprotein peak 1 (mol/mol protein) | peak 2 | peak 3 |
|---|---|---|---|
| Fucose | traces | 0 | 0 |
| Galactose | 0.3 | traces | 0.3 |
| Glucose | 2.1 | 2.1 | 2.1 |
| Mannose | 0.4 | 0.3 | 0.3 |

C) Biological characterization.

The biological activity is assayed by the methods of Example 2, items C.1.1, C.1.2, C.1.3 and the toxicity is determined according to C.2.

Inhibiting activity of protein synthesis with rabbit reticulocyte lysate (C.1.1):

| Fraction | Specific Activity (unit/mg x $10^{-3}$) | Extracted* Total Activity | Yield** (unit x x $10^{-6}$) |
|---|---|---|---|
| -"crude extract" | 7.9 | 183.5 | (100) |
| -"acid extract" | 16.3 | 221.0 | 120 |
| -"S-Sepharose percolate" | 35.3 | 266.5 | 145 |
| -"Sephacryl-S 200 eluate | 69.4 | 141.8 | 77 |
| -"CM-peak 1" | 89.9 | 15.7 | 8.5 |
| -"CM-peak 2" | 218,0 | 49.9 | 27.2 |
| -"asparin 1" | 112.4 | 6.1 | 3.3 |
| -"asparin 2" | 224.2 | 10.9 | 5.9 |

* : referred to 1 kg of seeds

** : referred to the "crude extract", assumed as 100.

IC50 for the protein synthesis in cell-free systems

Asparin 1:

| Method | Enzymatic systems | IC50 (nM) |
|---|---|---|
| C.1.1 | Rabbit reticulocyte lysate | 0.27 |
| C.1.2 | Purified ribosomes from reticulocytes | 8.8 |
| C.1.2 | Purified ribosomes from T. Brucei | > 3,330. |

Asparin 2:

| Method | Enzymatic systems | IC50 (nM) |
|---|---|---|
| C.1.1 | Rabbit reticulocyte lysate | 0.15 |
| C.1.2 | Purified ribosomes from reticulocytes | 6.9 |
| C.1.2 | Purified ribosomes from T. Brucei | > 3,330. |

IC50 for the protein synthesis in integer cells (C.1.3): (the values are the mean of two different determinations)

Asparin 1:

| Cells | ID50 (nM) |
|---|---|
| HeLa | 3,330 |
| TG | 610 |
| Jar | 3,330 |
| NB100 | 180 |
| Human fibroblasts | 3,330 |

Asparin 2:

| Cells | ID50 (nM) |
|---|---|
| HeLa | 3,330 |
| TG | 210 |
| Jar | 3,330 |
| NB100 | 180 |
| Human fibroblasts | 2,020 |

Toxicity in animals (C.2): LD50: 20 mg/kg body weight (both).

D) Chemical modifications

Chemical modifications are carried out as in Example 2, items D.1, D.2, D.3.

Determination of IC50 of modified asparin 1 and 2:

IC50 are determined according to the method C.1.1 of Example 2 and are expressed in nM. Each value is the mean of three determinations.

Asparin 1:

| Reagent | Reagent/RIP molar ratio | Number of introduced groups | IC50 (nM) |
|---|---|---|---|
| None | | | 0.27 |
| SPDP | 1.5 : 1 | 1.03 | 0.41 |
| SPDP | 2.0 : 1 | 1.32 | 0.67 |
| 2-Iminothiolane | 1.25 mM | 0.70 | 0.28 |
| SAMSA | 28 : 1 | 0.75 | 0.26 |

Asparin 2:

| Reagent | Reagent/RIP molar ratio | Number of introduced groups | IC50 (nM) |
|---|---|---|---|
| None | | | 0.15 |
| SPDP | 1.5 : 1 | 1.03 | 0.19 |
| SPDP | 2.0 : 1 | 1.32 | 0.40 |
| 2-Iminothiolane | 1.25 mM | 0.70 | 0.26 |
| SAMSA | 28 : 1 | 0.75 | 0.21 |

## EXAMPLE 5

Anti-HIV activity

Activity inhibiting the replication of HIVI in lymphoblastoid cultured cells.

VB cells, cultured under standard conditions, were inoculated with HIVI virus at known concentration for a period of about 60' at 37°C. The excess virus not bound to the cells is removed by washing and the cells are then incubated (about 1 X $10^5$ cells/ml) in the presence of increasing concentrations (between $10^{-8}$ and

$10^{-6}$M) of bryodine or asparin 1 or asparin 2 or momorcochin.

The presence of HIVI in the culture is determined by the analysis of the expression of the viral antigen p 24 using a commercial immunoassay.

The anti HIVI activity has been evaluated, as usual, in the period of the maximum virus production.

The inhibition of replication was evaluated as percent of the content of p 24 with respect to the controls.

The obtained results show that bryodine, asparin 1, asparin 2 and momorcochin are able to inhibit the virus replication at concentrations ($10^{-8}$ - $10^{-6}$ mols/l) not impairing the macromolecular synthesis of the cells, with inhibition values of 70-80%.

Inhibition of HIVI in infected monocyte machrophage cells

Monocyte/macrophage cells were isolated from peripheral blood from healthy volunteers according to the standard method with Ficoll gradient.

The cells were cultured after the HIVI infection as reported above.

Once the amount of infection was measured according to Crowe S., Mills J. e Mc Grath M. 1987 AIDS Res. Hum. Retrov. 3 135-145, the cells were treated with the RIPs of the invention at concentrations from $10^{-8}$ to $10^{-6}$ M and cultured for 4 days. The expression of the p 24 antigen was determined by cytofluorimetric analysis and the inhibition values expressed as percent versus controls. The results show that the RIPs of the invention can inhibit the virus replication at the tested concentrations, with inhibition values of 70-80%.

## Claims

**Claims for the following Contracting States : AT, BE, CH, DE, DK, FR, GB, IT, LI, LU, NL, SE**

**1.** A ribosome inactivating protein (RIP), having the following characteristics:

- relative molecular mass (Mr) by gel-filtration : 31.000
- relative molecular mass (Mr) by electrophoresis : 30.700
- isoelectric point : > 9
- extinction at 280 nm : 0.7
- amino acid composition for Mr 30.700

| aa | mol/mol pr. | aa | mol/mol pr. | aa | mol/mol pr. |
|---|---|---|---|---|---|
| Lys | : 15.1 | Glx | : 21.7 | Met | : 3.3 |
| His | : 2.7 | Pro | : 8.8 | Ile | : 12.3 |
| Arg | : 8.1 | Gly | : 11.3 | Leu | : 26.6 |
| Asx | : 27.7 | Ala | : 23.6 | Tyr | : 11.4 |
| Thr | : 18.4 | Half-Cys | :abs. | Phe | : 10.8 |
| Ser | : 19.2 | Val | : 18.7 | Trp | : abs. |

- total content in neutral sugars : 2.82%
- sugar composition (mols/mol RIP) for Mr 30.700

| Sugar | | mols/mol RIP |
|---|---|---|
| Fuc | : | 1.42 |
| Glc | : | 0.98 |
| Man | : | 2.16 |
| Xyl | : | 0.98 |
| Glc-$NH_2$ | : | abs. |

**2.** A ribosome inactivating protein, having the following characteristics:

- relative molecular mass (Mr) by gel-filtration : 27.300
- relative molecular mass (Mr) by electrophoresis : 28.800
- isoelectric point : > 9.5
- extinction at 280 nm : 0.8
- amino acid composition for Mr 28.800

| aa | mol/mol pr. | aa | mol/mol pr. | aa | mol/mol pr. |
|---|---|---|---|---|---|
| Lys | 10.8 | Glx | 18.9 | Met | 2.2 |
| His | 1.0 | Pro | 7.2 | Ile | 15.4 |
| Arg | 11.0 | Gly | 11.4 | Leu | 24.5 |
| Asx | 25.5 | Ala | 24.1 | Tyr | 11.7 |
| Thr | 17.4 | Half-Cys | abs. | Phe | 7.4 |
| Ser | 24.4 | Val | 14.4 | Trp | abs. |

- total content in neutral sugars : 2.72%
- sugar composition (mols/mol RIP) for Mr 28.800

| Sugar | | mols/mol RIP |
|---|---|---|
| Fuc | : | 1.52 |
| Glc | : | 0.43 |
| Man | : | 2.52 |
| Xyl | : | 0.63 |

**3.** A ribosome inactivating protein, having the following characteristics:

- relative molecular mass (Mr) by gel-filtration : 29.700
- relative molecular mass (Mr) by electrophoresis : 30.500
- isoelectric point : > 8.7
- extinction at 280 nm : 1.0
- amino acid composition for Mr 30.500

| aa | mol/mol pr. | aa | mol/mol pr. | aa | mol/mol pr. |
|---|---|---|---|---|---|
| Lys | : 15.3 | Glx | : 24.3 | Met | : 4.1 |
| His | : 3.4 | Pro | : 14.9 | Ile | : 11.4 |
| Arg | : 14.4 | Gly | : 15.0 | Leu | : 26.3 |
| Asx | : 26.5 | Ala | : 19.5 | Tyr | : 9.8 |
| Thr | : 13.9 | Half-Cys | : 3.3 | Phe | : 6.3 |
| Ser | : 11.0 | Val | : 16.7 | Trp | : abs. |

**4.** A ribosome inactivating protein, having the following characteristics:

- relative molecular mass (Mr) by gel-filtration : 28.100
- relative molecular mass (Mr) by electrophoresis : 29.800
- isoelectric point : > 9.2
- extinction at 280 nm : 1.0
- amino acid composition for Mr 29.800

| aa | mol/mol pr. | aa | mol/mol pr. | aa | mol/mol pr. |
|---|---|---|---|---|---|
| Lys | : 14.9 | Glx | : 23.2 | Met | : 1.1 |
| His | : 2.9 | Pro | : 15.4 | Ile | : 11.5 |
| Arg | : 14.8 | Gly | : 15.2 | Leu | : 26.6 |
| Asx | : 27.2 | Ala | : 18.8 | Tyr | : 9.8 |
| Thr | : 14.2 | Half-Cys | : 1.3 | Phe | : 6.3 |
| Ser | : 11.3 | Val | : 16.9 | Trp | : abs. |

5. Protein derivatives as claimed in claims 1 to 4, having formula (I):

$$Tox ---- \left[ ---- Y ---- S ---- R \right]_x \qquad (I)$$

wherein Tox represents the ribosome inactivating protein of claims 1 - 4, Y is selected from the group consisting of

$-NHCOCH_2CH_2-$; 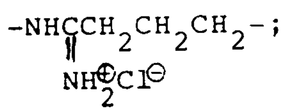

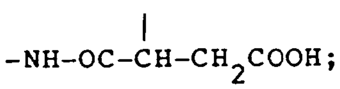

R is selected from hydrogen, acetyl, 2-pyridyl and x is comprised from 0.2 to 3.

**Claims for the following Contracting States : GR, ES**

1. A process for the preparation of the ribosome inactivating protein (RIP), named momorcochin from Momordica cochinchinensis seeds having the following characteristic:
   - relative molecular mass (Mr) by gel-filtration : 31.000
   - relative molecular mass (Mr) by electrophoresis : 30.700
   - isoelectric point : > 9
   - extinction at 280 nm : 0.7
   - amino acid composition for Mr 30.700

| aa | mol/mol pr. | aa | mol/mol pr. | aa | mol/mol pr. |
|---|---|---|---|---|---|
| Lys | : 15.1 | Glx | : 21.7 | Met | : 3.3 |
| His | : 2.7 | Pro | : 8.8 | Ile | : 12.3 |
| Arg | : 8.1 | Gly | : 11.3 | Leu | : 26.6 |
| Asx | : 27.7 | Ala | : 23.6 | Tyr | : 11.4 |
| Thr | : 18.4 | Half-Cys | :abs. | Phe | : 10.8 |
| Ser | : 19.2 | Val | : 18.7 | Trp | : abs. |

- total content in neutral sugars : 2.82%
- sugar composition (mols/mol RIP) for Mr 30.700

| Sugar | | mols/mol RIP |
|---|---|---|
| Fuc | : | 1.42 |
| Glc | : | 0.98 |
| Man | : | 2.16 |
| Xyl | : | 0.98 |
| $Glc-NH_2$ | : | abs. |

comprising:

a) grinding and extraction of seeds of Momordica cochinchinensis, recovery of the extracts,;
b) centrifugation of the extracts of step a); recovery of the crude extract;
c) acidification of the crude extract to pH 4.2-4.8;
d) chromatography of the acidic extract on a S-Sepharose(R) column;
e) chromatography of the eluate from step d) on a Sephadex-G-50(R) column and subsequent chromatography of the eluate therefrom on a CM-Sepharose Fast Flow(R) column;
f) final purification by means of dialysis, lyophilization, chromatography, precipitation or other usual methods.

**2.** A process for the preparation of the ribosome inactivating protein (RIP), named bryodine-L from Bryonia dioica leaves having the following characteristics:

- relative molecular mass (Mr) by gel-filtration : 27.300
- relative molecular mass (Mr) by electrophoresis : 28.800
- isoelectric point : > 9.5
- extinction at 280 nm : 0.8
- amino acid composition for Mr 28.800

| aa | mol/mol pr. | aa | mol/mol pr. | aa | mol/mol pr. |
|---|---|---|---|---|---|
| Lys | : 10.8 | Glx | : 18.9 | Met | : 2.2 |
| His | : 1.0 | Pro | : 7.2 | Ile | : 15.4 |
| Arg | : 11.0 | Gly | : 11.4 | Leu | : 24.5 |
| Asx | : 25.5 | Ala | : 24.1 | Tyr | : 11.7 |
| Thr | : 17.4 | Half-Cys | : abs. | Phe | : 7.4 |
| Ser | : 24.4 | Val | : 14.4 | Trp | : abs. |

- total content in neutral sugars : 2.72% sugar
- composition (mols/mol RIP) for Mr 28.800

| Sugar | mols/mol RIP |
|---|---|
| Fuc | : 1.52 |
| Glc | : 0.43 |
| Man | : 2.52 |
| Xyl | : 0.63 |

comprising:

a) extraction of the leaves of Bryonia dioica, recovery of the extracts;
b) centrifugation of the extracts of step a); recovery of the crude extract;
c) acidification of the crude extract to pH 4.2-4.8;
d) chromatography of the acidic extract on a S-Sepharose(R) column;
e) chromatography of the eluate from step d) on a Sephacryl-S-200(R) column and subsequent chromatography of the eluate therefrom on a CM-Sepharose Fast Flow(R) column;
f) final purification by means of dialysis, lyophilization, chromatography, precipitation or other usual methods.

**3.** A process for the preparation of the ribosome inactivating proteins (RIP), named asparin 1 and asparin 2 from Asparagus officinalis seeds having the following characteristics, respectively:
asparin 1

- relative molecular mass (Mr) by gel-filtration : 29.700
- relative molecular mass (Mr) by electrophoresis : 30.500
- isoelectric point : > 8.7
- extinction at 280 nm : 1.0
- amino acid composition for Mr 30.500

| aa | mol/mol pr. | aa | mol/mol pr. | aa | mol/mol pr. |
|---|---|---|---|---|---|
| Lys | : 15.3 | Glx | : 24.3 | Met | : 4.1 |
| His | : 3.4 | Pro | : 14.9 | Ile | : 11.4 |
| Arg | : 14.4 | Gly | : 15.0 | Leu | : 26.3 |
| Asx | : 26.5 | Ala | : 19.5 | Tyr | : 9.8 |
| Thr | : 13.9 | Half-Cys | : 3.3 | Phe | : 6.3 |
| Ser | : 11.0 | Val | : 16.7 | Trp | : abs. |

asparin 2:

- relative molecular mass (Mr) by gel-filtration : 28.100
- relative molecular mass (Mr) by electrophoresis : 29.800
- isoelectric point : > 9.2
- extinction at 280 nm : 1.0
- amino acid composition for Mr 29.800

| aa | mol/mol pr. | aa | mol/mol pr. | aa | mol/mol pr. |
|---|---|---|---|---|---|
| Lys | : 14.9 | Glx | : 23.2 | Met | : 1.1 |
| His | : 2.9 | Pro | : 15.4 | Ile | : 11.5 |
| Arg | : 14.8 | Gly | : 15.2 | Leu | : 26.6 |
| Asx | : 27.2 | Ala | : 18.8 | Tyr | : 9.8 |
| Thr | : 14.2 | Half-Cys | : 1.3 | Phe | : 6.3 |
| Ser | : 11.3 | Val | : 16.9 | Trp | : abs. |

comprising:

a) grinding and extraction of seeds of Asparagus officinalis, recovery the extracts;
b) centrifugation of the extract of step a); recovery the crude extract;
c) adjusting to pH 4.2-4.8, obtaining an acidic extract;
d) chromatography of the acidic extract on a S-Sepharose(R) column;
e) chromatography of the eluate from step d) on a Sephadex-G-50(R) column and subsequent chromatography of the eluate therefrom on a CM-Sepharose Fast Flow (R) column, obtaining two fractions of asparin 1 and asparin 2, respectively;
f) final purification by means of dialysis, lyophilization, chromatography, precipitation or other usual methods.

**4.** A process for the preparation of derivatives of the proteins according to claims 1 to 3, having formula (I):

$$\text{Tox} \text{----} \left[ \text{----Y----S----R} \right]_x \qquad (I)$$

wherein Tox represents the ribosome inactivating protein of claims 1-3, Y is selected from the group consisting of

$$-NHCOCH_2CH_2-; \quad -NH\underset{\underset{NH_2^{\oplus}Cl^{\ominus}}{\|}}{C}CH_2CH_2CH_2-;$$

$$-NH-OC-\overset{|}{C}H-CH_2COOH;$$

R is selected from hydrogen, acetyl, 2-pyridyl and x is ranging from 0.2 to 3, comprising the reaction between the protein and a reagent selected from the group consisting in N-succinimidyl-3-(2-pyridylthiopropionate (SPDP), 2-iminothiolane, S-acetylmercaptosuccinic anhydride.

**Patentansprüche**

**Patentansprüche für folgende Vertragsstaaten : AT, BE, CH, DE, DK, FR, GB, IT, LI, LU, NL, SE**

1. Ribosom-inaktivierendes Protein (RIP), das die folgenden Eigenschaften aufweist:
   - relative Molekularmasse (Mr), bestimmt durch Gelfiltration: 31 000
   - relative Molekularmasse (Mr) bestimmt durch Elektrophorese: 30 700
   - isoelektrischer Punkt: > 9
   - Extinktion bei 280 nm: 0,7
   - Aminosäurezusammensetzung für Mr 30 700

| aa | mol/mol Protein | aa | mol/mol Protein | aa | mol/mol Protein |
|---|---|---|---|---|---|
| Lys: | 15,1 | Glx: | 21,7 | Met: | 3,3 |
| His: | 2,7 | Pro: | 8,8 | Ile: | 12,3 |
| Arg: | 8,1 | Gly: | 11,3 | Leu: | 26,6 |
| Asx: | 27,7 | Ala: | 23,6 | Tyr: | 11,4 |
| Thr: | 18,4 | Half-Cys: | abs. | Phe: | 10,8 |
| Ser: | 19,2 | Val: | 18,7 | Trp: | abs. |

   - Gesamtgehalt an neutralen Zuckern: 2,82 %
   - Zuckerzusammensetzung (mole/mol RIP) für Mr 30 700

| Zucker | mole/mol RIP |
|---|---|
| Fuc: | 1,42 |
| Glc: | 0,98 |
| Man: | 2,16 |
| Xyl: | 0,98 |
| $Glc\text{-}NH_2$ | abs. |

2. Ribosom-inaktivierendes Protein, das die folgenden Eigenschaften aufweist:
   - relative Molekularemasse (Mr), bestimmt durch Gelfiltration: 27 300
   - relative Molekularmasse (Mr) bestimmt durch Elektrophorese: 28 800
   - isoelektrischer Punkt: > 9,5
   - Extinktion bei 280 nm: 0,8
   - Aminosäurezusammensetzung für Mr 28 800

| aa | mol/mol Protein | aa | mol/mol Protein | aa | mol/mol Protein |
|---|---|---|---|---|---|
| Lys: | 10,8 | Glx: | 18,9 | Met: | 2,2 |
| His: | 1,0 | Pro: | 7,2 | Ile: | 15,4 |
| Arg: | 11,0 | Gly: | 11,4 | Leu: | 24,5 |
| Asx: | 25,5 | Ala: | 24,1 | Tyr: | 11,7 |
| Thr: | 17,4 | Half-Cys: | abs. | Phe: | 7,4 |
| Ser: | 24,4 | Val: | 14,4 | Trp: | abs. |

   - Gesamtgehalt an neutralen Zuckern: 2,72 %
   - Zuckerzusammensetzung (mole/mol RIP) für Mr 28 800

| Zucker | mole/mol RIP |
|---|---|
| Fuc: | 1,52 |
| Glc: | 0,43 |
| Man: | 2,52 |
| Xyl: | 0,63 |

3. Ribosom-inaktivierendes Protein, das die folgenden Eigenschaften aufweist:
   - relative Molekularmasse (Mr), bestimmt durch Gelfiltration: 29 700
   - relative Molekularmasse (Mr) bestimmt durch Elektrophorese: 30 500
   - isoelektrischer Punkt: > 8,7
   - Extinktion bei 280 nm: 1,0

- Aminosäurezusammensetzung für Mr 30 500

| aa | mol/mol Protein | aa | mol/mol Protein | aa | mol/mol Protein |
|---|---|---|---|---|---|
| Lys: | 15,3 | Glx: | 24,3 | Met: | 4,1 |
| His: | 3,4 | Pro: | 14,9 | Ile: | 11,4 |
| Arg: | 14,4 | Gly: | 15,0 | Leu: | 26,3 |
| Asx: | 26,5 | Ala: | 19,5 | Tyr: | 9,8 |
| Thr: | 13,9 | Half-Cys: | 3,3 | Phe: | 6,3 |
| Ser: | 11,0 | Val: | 16,7 | Trp: | abs. |

4. Ribosom-inaktivierendes Protein, das die folgenden Eigenschaften aufweist:
   - relative Molekularmasse (Mr), bestimmt durch Gelfiltration: 28 100
   - relative Molekularmasse (Mr) bestimmt durch Elektrophorese: 29 800
   - isoelektrischer Punkt: > 9,2
   - Extinktion bei 280 nm: 1,0
   - Aminosäurezusammensetzung für Mr 29 800

| aa | mol/mol Protein | aa | mol/mol Protein | aa | mol/mol Protein |
|---|---|---|---|---|---|
| Lys: | 14,9 | Glx: | 23,2 | Met: | 1,1 |
| His: | 2,9 | Pro: | 15,4 | Ile: | 11,5 |
| Arg: | 14,8 | Gly: | 15,2 | Leu: | 26,6 |
| Asx: | 27,2 | Ala: | 18,8 | Tyr: | 9,8 |
| Thr: | 14,2 | Half-Cys: | 1,3 | Phe: | 6,3 |
| Ser: | 11,3 | Val: | 16,9 | Trp: | abs. |

5. Protein-Derivate nach einem der Ansprüche 1 bis 4, welche die Formel (I) haben:

$$(I):\quad \text{Tox} ---- \left[ ---- Y ---- S ---- R \right]_x \qquad (I)$$

worin:

Tox steht für das Ribosom-inaktivierende Protein nach den Ansprüchen 1 bis 4,
Y ausgewählt wird aus der Gruppe, die besteht aus

$-NHCOCH_2CH_2-$; 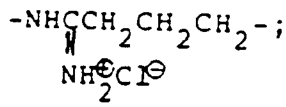

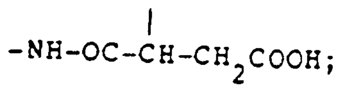

R ausgewählt wird aus Wasserstoff, Acetyl, 2-Pyridyl und
x steht für 0,2 bis 3.

**Patentansprüche für folgende Vertragsstaaten : ES, GR**

1. Verfahren zur Herstellung des Ribosom-inaktivierenden Proteins (RIP), als Momorcochin bezeichnet, aus Momordica cochinchinensis-Samen, das die folgenden Eigenschaften hat:
   - relative Molekularmasse (Mr), bestimmt durch Gelfiltration: 31 000
   - relative Molekularmasse (Mr) bestimmt durch Elektrophorese: 30 700
   - isoelektrischer Punkt: > 9
   - Extinktion bei 280 nm: 0,7
   - Aminosäurezusammensetzung für Mr 30 700

| aa | mol/mol Protein | aa | mol/mol Protein | aa | mol/mol Protein |
|---|---|---|---|---|---|
| Lys: | 15,1 | Glx: | 21,7 | Met: | 3,3 |
| His: | 2,7 | Pro: | 8,8 | Ile: | 12,3 |
| Arg: | 8,1 | Gly: | 11,3 | Leu: | 26,6 |
| Asx: | 27,7 | Ala: | 23,6 | Tyr: | 11,4 |
| Thr: | 18,4 | Half-Cys: | abs. | Phe: | 10,8 |
| Ser: | 19,2 | Val: | 18,7 | Trp: | abs. |

   - Gesamtgehalt an neutralen Zuckern: 2,82 %
   - Zuckerzusammensetzung (mole/mol RIP) für Mr 30 700

| Zucker | mole/mol RIP |
|---|---|
| Fuc: | 1,42 |
| Glc: | 0,98 |
| Man: | 2,16 |
| Xyl: | 0,98 |
| Glc-$NH_2$ | abs. |

das umfaßt:

a) das Mahlen und Extrahieren der Samen von Momordica cochinchinensis und die Gewinnung der Extrakte;
b) das Zentrifugieren der Extrakte der Stufe (a) und die Gewinnung des rohen Extrakts;
c) das Ansäuern des rohen Extrakts auf pH 4,2 bis 4,8;
d) das Chromatographieren des sauren Extrakts an einer S-Sepharose ®-Säule;
e) das Chromatographieren des Eluats aus der Stufe (d) an einer Sephadex-G-50 ®-Säule und das anschließende Chromatographieren des Eluats daraus an einer CM-Sepharose-Fast Flow ®-Säule;
f) und schließlich die Reinigung mittels Dialyse-, Lyophilisierungs-, Chromatographie-, Fällungs- oder anderer üblicher Methoden.

**2.** Verfahren zur Herstellung des Ribosom-inaktivierenden Proteins, (RIP), als Bryodine-L bezeichnet, aus Bryonia dioica-Blättern, das die folgenden Eigenschaften aufweist:

- relative Molekularemasse (Mr), bestimmt durch Gelfiltration: 27 300
- relative Molekularmasse (Mr) bestimmt durch Elektrophorese: 28 800
- isoelektrischer Punkt: > 9,5
- Extinktion bei 280 nm: 0,8
- Aminosäurezusammensetzung für Mr 28 800

| aa | mol/mol Protein | aa | mol/mol Protein | aa | mol/mol Protein |
|---|---|---|---|---|---|
| Lys: | 10,8 | Glx: | 18,9 | Met: | 2,2 |
| His: | 1,0 | Pro: | 7,2 | Ile: | 15,4 |
| Arg: | 11,0 | Gly: | 11,4 | Leu: | 24,5 |
| Asx: | 25,5 | Ala: | 24,1 | Tyr: | 11,7 |
| Thr: | 17,4 | Half-Cys: | abs. | Phe: | 7,4 |
| Ser: | 24,4 | Val: | 14,4 | Trp: | abs. |

- Gesamtgehalt an neutralen Zuckern: 2,72 %
- Zuckerzusammensetzung (mole/mol RIP) für Mr 28 800

| Zucker | mole/mol RIP |
|---|---|
| Fuc: | 1,52 |
| Glc: | 0,43 |
| Man: | 2,52 |
| Xyl: | 0,63 |

das umfaßt:

a) die Extraktion der Blätter von Bryonia dioica und die Gewinnung der Extrakte;
b) das Zentrifugieren der Extrakte der Stufe (a) und die Gewinnung des rohen Extrakts;
c) das Ansäuern des rohen Extrakts auf pH 4,2 bis 4,8;
d) das Chromatographieren des sauren Extrakts an einer S-Sepharose ®-Säule;
e) das Chromatographieren des Eluats aus der Stufe (d) an einer Sephacryl-S-200 ®-Säule und das anschließende Chromatographieren des Eluats daraus an einer CM-Sepharose-Fast Flow ®-Säule;
f) und schließlich das Reinigen mittels Dialyse-, Lyophilisierungs-, Chromatographie-, Fällungs- oder anderer üblicher Methoden.

3. Verfahren zur Herstellung der Ribosom-inaktivierenden Proteine (RIP), als Asparin 1 und Asparin 2 bezeichnet, aus Asparagus officinalis-Samen, die jeweils die folgenden Eigenschaften aufweisen:

Asparin 1

- relative Molekularmasse (Mr), bestimmt durch Gelfiltration: 29 700
- relative Molekularmasse (Mr) bestimmt durch Elektrophorese: 30 500
- isoelektrischer Punkt: > 8,7
- Extinktion bei 280 nm: 1,0
- Aminosäurezusammensetzung für Mr 30 500

| aa | mol/mol Protein | aa | mol/mol Protein | aa | mol/mol Protein |
|---|---|---|---|---|---|
| Lys: | 15,3 | Glx: | 24,3 | Met: | 4,1 |
| His: | 3,4 | Pro: | 14,9 | Ile: | 11,4 |
| Arg: | 14,4 | Gly: | 15,0 | Leu: | 26,3 |
| Asx: | 26,5 | Ala: | 19,5 | Tyr: | 9,8 |
| Thr: | 13,9 | Half-Cys: | 3,3 | Phe: | 6,3 |
| Ser: | 11,0 | Val: | 16,7 | Trp: | abs. |

Asparin 2

- relative Molekularmasse (Mr), bestimmt durch Gelfiltration: 28 100
- relative Molekularmasse (Mr) bestimmt durch Elektrophorese: 29 800
- isoelektrischer Punkt: > 9,2
- Extinktion bei 280 nm: 1,0
- Aminosäurezusammensetzung für Mr 29 800

| aa | mol/mol Protein | aa | mol/mol Protein | aa | mol/mol Protein |
|---|---|---|---|---|---|
| Lys: | 14,9 | Glx: | 23,2 | Met: | 1,1 |
| His: | 2,9 | Pro: | 15,4 | Ile: | 11,5 |
| Arg: | 14,8 | Gly: | 15,2 | Leu: | 26,6 |
| Asx: | 27,2 | Ala: | 18,8 | Tyr: | 9,8 |
| Thr: | 14,2 | Half-Cys: | 1,3 | Phe: | 6,3 |
| Ser: | 11,3 | Val: | 16,9 | Trp: | abs. |

das umfaßt:

a) das Mahlen und Extrahieren der Samen von Asparagus officinalis und die Gewinnung der Extrakte;
b) das Zentrifugieren des Extrakts der Stufe (a) und die Gewinnung des rohen Extrakts;
c) die Einstellung auf pH 4,2 bis 4,8 unter Bildung eines sauren Extrakts;
d) das Chromatographieren des sauren Extrakts an einer S-Sepharose ®-Säule;
e) das Chromatographieren des Eluats aus der Stufe (d) an einer Sephadex-G-50 ®-Säule und das anschließende Chromatographieren des Eluats daraus an einer CM-Sepharose-Fast Flow ®-Säule, wobei man zwei Fraktionen von Asparin 1 bzw. Asparin 2 erhält;
f) und schließlich das Reinigen mittels Dialyse-, Lyophilisierungs-, Chromatographie-, Fällungs- oder anderer üblicher Methoden.

**4.** Verfahren zur Herstellung von Derivaten der Proteine nach den Ansprüchen 1 bis 3, welche die Formel (I) haben:

$$Tox ---- \left[ ----Y----S----R \right]_x \qquad (I)$$

worin:

Tox steht für das Ribosom-inaktivierende Protein der Ansprüche 1 bis 3,
Y ausgewählt wird aus der Gruppe, die besteht aus

$-NHCOCH_2CH_2-$; 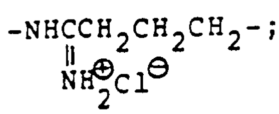

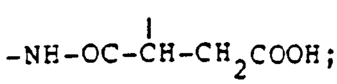

R ausgewält wird aus Wasserstoff, Acetyl, 2-Pyridyl und
x steht für 0,2 bis 3,

das umfaßt die Umsetzung des Proteins mit einem Reagens, ausgewählt aus der Gruppe, die besteht aus N-Succinimidyl-3-(2-pyridyl)thiopropionat (SPDP), 2-Iminothiolan und S-Acetylmercaptobernsteinsäure-anhydrid.

## Revendications

**Revendications pour les Etats contractants suivants : AT, BE, CH, DE, DK, FR, GB, IT, LI, LU, NL, SE**

**1.** Une protéine inactivant les ribosomes (RIP) ayant les caractéristiques suivantes :

- masse moléculaire relative (Mr) par filtration sur gel : 31 000
- masse moléculaire relative (Mr) par électrophorèse : 30 700
- point isoélectrique : >9
- extinction à 280 nm : 0,7
- composition en acides aminés pour Mr 30 700

| aa | mol/mol de protéine | aa | mol/mol de protéine | aa | mol/mol de protéine |
|---|---|---|---|---|---|
| Lys : | 15,1 | Glx : | 21,7 | Met : | 3,3 |
| His : | 2,7 | Pro : | 8,8 | Ile : | 12,3 |
| Arg : | 8,1 | Gly : | 11,3 | Leu : | 26,6 |
| Asx : | 27,7 | Ala : | 23,6 | Tyr : | 11,4 |
| Thr : | 18,4 | Hémi-Cys : | absent | Phe : | 10,8 |
| Ser : | 19,2 | Val : | 18,7 | Trp : | absent |

- teneur totale en sucres neutres : 2,82 %
- composition en sucres (mol/mol de RIP) pour Mr 30 700

| Sucre | mol/mol de RIP |
|---|---|
| Fuc : | 1,42 |
| Glc : | 0,98 |
| Man : | 2,16 |
| Xyl : | 0,98 |
| Glc-$NH_2$ : | absent |

**2.** Une protéine inactivant les ribosomes (RIP) ayant les caractéristiques suivantes :

- masse moléculaire relative (Mr) par filtration sur gel : 27 300
- masse moléculaire relative (Mr) par électrophorèse : 28 800
- point isoélectrique : >9,5
- extinction à 280 nm : 0,8
- composition en acides aminés pour Mr 28 800

| aa | mol/mol de protéine | aa | mol/mol de protéine | aa | mol/mol de protéine |
|---|---|---|---|---|---|
| Lys | : 10,8 | Glx | : 18,9 | Met | : 2,2 |
| His | : 1,0 | Pro | : 7,2 | Ile | : 15,4 |
| Arg | : 11,0 | Gly | : 11,4 | Leu | : 24,5 |
| Asx | : 25,5 | Ala | : 24,1 | Tyr | : 11,7 |
| Thr | : 17,4 | Hémi-Cys | : absent | Phe | : 7,4 |
| Ser | : 24,4 | Val | : 14,4 | Trp | : absent |

- teneur totale en sucres neutres : 2,72 %
- composition en sucres (mol/mol de RIP) pour Mr 28 800

| Sucre | | mol/mol de RIP |
|---|---|---|
| Fuc | : | 1,52 |
| Glc | : | 0,43 |
| Man | : | 2,52 |
| Xyl | : | 0,63 |

**3.** Une protéine inactivant les ribosomes (RIP) ayant les caractéristiques suivantes :
- masse moléculaire relative (Mr) par filtration sur gel : 29 700
- masse moléculaire relative (Mr) par électrophorèse : 30 500
- point isoélectrique : >8,7
- extinction à 280 nm : 1,0
- composition en acides aminés pour Mr 30 500

| aa | mol/mol de protéine | aa | mol/mol de protéine | aa | mol/mol de protéine |
|---|---|---|---|---|---|
| Lys | : 15,3 | Glx | : 24,3 | Met | : 4,1 |
| His | : 3,4 | Pro | : 14,9 | Ile | : 11,4 |
| Arg | : 14,4 | Gly | : 15,0 | Leu | : 26,3 |
| Asx | : 26,5 | Ala | : 19,5 | Tyr | : 9,8 |
| Thr | : 13,9 | Hémi-Cys | : 3,3 | Phe | : 6,3 |
| Ser | : 11,0 | Val | : 16,7 | Trp | : absent |

**4.** Une protéine inactivant les ribosomes (RIP) ayant les caractéristiques suivantes :
- masse moléculaire relative (Mr) par filtration sur gel : 28 100
- masse moléculaire relative (Mr) par électrophorèse : 29 800
- point isoélectrique : >9,2
- extinction à 280 nm : 1,0
- composition en acides aminés pour Mr 29 800

| aa | mol/mol de protéine | aa | mol/mol de protéine | aa | mol/mol de protéine |
|---|---|---|---|---|---|
| Lys | : 14,9 | Glx | : 23,2 | Met | : 1,1 |
| His | : 2,9 | Pro | : 15,4 | Ile | : 11,5 |
| Arg | : 14,8 | Gly | : 15,2 | Leu | : 26,6 |
| Asx | : 27,2 | Ala | : 18,8 | Tyr | : 9,8 |
| Thr | : 14,2 | Hémi-Cys | : 1,3 | Phe | : 6,3 |
| Ser | : 11,3 | Val | : 16,9 | Trp | : absent |

5. Dérivés des protéines selon les revendications 1 à 4, répondant à la formule (I) :

$$Tox ---- \left[ ----Y----S----R \right]_x \qquad (I)$$

où Tox représente la protéine inactivant les ribosomes des revendications 1 à 4, Y est choisi dans le groupe formé par

$-NHCOCH_2CH_2-$; 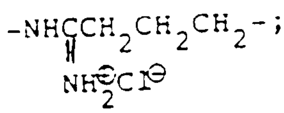

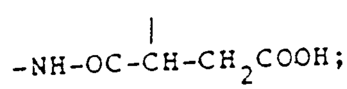

R est choisi parmi l'hydrogène, le groupe acétyle, le groupe 2-pyridyle, et x est compris entre 0,2 et 3.

**Revendications pour les Etats contractants suivants : GR, ES**

1. Un procédé pour la préparation de la protéine inactivant les ribosomes (RIP) nommée momorcochine à partir de graines de *Momordica cochinchinensis*, ayant les caractéristiques suivantes :
   - masse moléculaire relative (Mr) par filtration sur gel : 31 000
   - masse moléculaire relative (Mr) par électrophorèse : 30 700
   - point isoélectrique : >9
   - extinction à 280 nm : 0,7
   - composition en acides aminés pour Mr 30 700

| aa | mol/mol de protéine | aa | mol/mol de protéine | aa | mol/mol de protéine |
|---|---|---|---|---|---|
| Lys | 15,1 | Glx | 21,7 | Met | 3,3 |
| His | 2,7 | Pro | 8,8 | Ile | 12,3 |
| Arg | 8,1 | Gly | 11,3 | Leu | 26,6 |
| Asx | 27,7 | Ala | 23,6 | Tyr | 11,4 |
| Thr | 18,4 | Hémi-Cys | absent | Phe | 10,8 |
| Ser | 19,2 | Val | 18,7 | Trp | absent |

- teneur totale en sucres neutres : 2,82 %
- composition en sucres (mol/mol de RIP) pour Mr 30 700

| Sucre | mol/mol de RIP |
|---|---|
| Fuc | 1,42 |
| Glc | 0,98 |
| Man | 2,16 |
| Xyl | 0,98 |
| Glc-$NH_2$ | absent |

comprenant les étapes de :

a) broyage et extraction de graines de *Momordica cochinchinensis* ; collecte des extraits ;
b) centrifugation des extraits de l'étape a) ; collecte de l'extrait brut ;
c) acidification de l'extrait brut jusqu'à pH 4,2-4,8 ;
d) chromatographie de l'extrait acide sur une colonne de S-Sepharose® ;
e) chromatographie de l'éluat obtenu à l'étape d) sur une colonne de Sephadex-G-50® et chromatographie subséquente de l'éluat qui en provient sur une colonne de CM-Sepharose Fast Flow® ;
f) purification finale par dialyse, lyophilisation, chromatographie, précipitation ou d'autres techniques usuelles.

**2.** Un procédé pour la préparation de la protéine inactivant les ribosomes (RIP) nommée bryodine-L à partir de feuilles de *Bryonia dioica*, ayant les caractéristiques suivantes :

- masse moléculaire relative (Mr) par filtration sur gel : 27 300
- masse moléculaire relative (Mr) par électrophorèse : 28 800
- point isoélectrique : >9,5
- extinction à 280 nm : 0,8
- composition en acides aminés pour Mr 28 800

| aa | mol/mol de protéine | aa | mol/mol de protéine | aa | mol/mol de protéine |
|---|---|---|---|---|---|
| Lys | : 10,8 | Glx | : 18,9 | Met | : 2,2 |
| His | : 1,0 | Pro | : 7,2 | Ile | : 15,4 |
| Arg | : 11,0 | Gly | : 11,4 | Leu | : 24,5 |
| Asx | : 25,5 | Ala | : 24,1 | Tyr | : 11,7 |
| Thr | : 17,4 | Hémi-Cys | : absent | Phe | : 7,4 |
| Ser | : 24,4 | Val | : 14,4 | Trp | : absent |

- teneur totale en sucres neutres : 2,72 %
- composition en sucres (mol/mol de RIP) pour Mr 28 800

| Sucre | | mol/mol de RIP |
|---|---|---|
| Fuc | : | 1,52 |
| Glc | : | 0,43 |
| Man | : | 2,52 |
| Xyl | : | 0,63 |

comprenant les étapes de :

a) extraction des feuilles de *Bryonia dioica* ; collecte des extraits ;
b) centrifugation des extraits de l'étape a) ; collecte de l'extrait brut ;
c) acidification de l'extrait brut jusqu'à pH 4,2-4,8 ;
d) chromatographie de l'extrait acide sur une colonne de S-Sepharose® ;
e) chromatographie de l'éluat obtenu à l'étape d) sur une colonne de Sephacryl-S-200® et chromatographie subséquente de l'éluat qui en provient sur une colonne de CM-Sepharose Fast Flow® ;
f) purification finale par dialyse, lyophilisation, chromatographie, précipitation ou d'autres techniques usuelles.

**3.** Un procédé pour la préparation des protéines inactivant les ribosomes (RIP) nommées asparine 1 et asparine 2 à partir de graines de *Asparagus officinalis*, ayant respectivement les caractéristiques suivantes :
asparine 1 :

- masse moléculaire relative (Mr) par filtration sur gel : 29 700
- masse moléculaire relative (Mr) par électrophorèse : 30 500
- point isoélectrique : >8,7
- extinction à 280 nm : 1,0
- composition en acides aminés pour Mr 30 500

| aa mol/mol de protéine | aa mol/mol de protéine | aa mol/mol de protéine |
|---|---|---|
| Lys : 15,3 | Glx : 24,3 | Met : 4,1 |
| His : 3,4 | Pro : 14,9 | Ile : 11,4 |
| Arg : 14,4 | Gly : 15,0 | Leu : 26,3 |
| Asx : 26,5 | Ala : 19,5 | Tyr : 9,8 |
| Thr : 13,9 | Hémi-Cys : 3,3 | Phe : 6,3 |
| Ser : 11,0 | Val : 16,7 | Trp : absent |

asparine 2 :

- masse moléculaire relative (Mr) par filtration sur gel : 28 100
- masse moléculaire relative (Mr) par électrophorèse : 29 800
- point isoélectrique : >9,2
- extinction à 280 nm : 1,0
- composition en acides aminés pour Mr 29 800

| aa mol/mol de protéine | aa mol/mol de protéine | aa mol/mol de protéine |
|---|---|---|
| Lys : 14,9 | Glx : 23,2 | Met : 1,1 |
| His : 2,9 | Pro : 15,4 | Ile : 11,5 |
| Arg : 14,8 | Gly : 15,2 | Leu : 26,6 |
| Asx : 27,2 | Ala : 18,8 | Tyr : 9,8 |
| Thr : 14,2 | Hémi-Cys : 1,3 | Phe : 6,3 |
| Ser : 11,3 | Val : 16,9 | Trp : absent |

comprenant les étapes de :

a) broyage et extraction de graines de *Asparagus officinalis* ; collecte des extraits ;
b) centrifugation des extraits de l'étape a) ; collecte de l'extrait brut ;
c) ajustement à pH 4,2-4,8 pour l'obtention d'un extrait acide ;
d) chromatographie de l'extrait acide sur une colonne de S-Sepharose® ;
e) chromatographie de l'éluat obtenu à l'étape d) sur une colonne de Sephadex-G-50® et chromatographie subséquente de l'éluat qui en provient sur une colonne de CM-Sepharose Fast Flow® ; obtention de deux fractions d'asparine 1 et d'asparine 2, respectivement ;
f) purification finale par dialyse, lyophilisation, chromatographie, précipitation ou d'autres techniques usuelles.

**4.** Un procédé pour la préparation de dérivés des protéines selon les revendications 1 à 3, répondant à la formule (I) :

$$Tox ---- \left[ ----Y----S----R \right]_x \qquad (I)$$

où Tox représente la protéine inactivant les ribosomes des revendications 1 à 3, Y est choisi dans le groupe formé par

$$-NHCOCH_2CH_2-;$$ 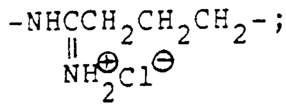

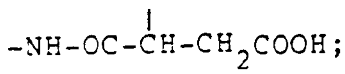

R est choisi parmi l'hydrogène, le groupe acétyle, le groupe 2-pyridyle, et x est compris entre 0,2 et 3, comprenant la réaction entre la protéine et un réactif choisi dans le groupe formé par le N-succinimidyl-3-(2-pyridylthiopropionate (SPDP), le 2-iminothiolane, l'anhydride S-acétylmercaptosuccinique.